# EUROPÄISCHE PATENTSCHRIFT

(11) **EP 0 960 608 B1**
(45) Veröffentlichungstag und Bekanntmachung des Hinweises auf die Patenterteilung: **12.01.2005**
(21) Anmeldenummer: 99810455.8
(22) Anmeldetag: 26.05.1999
(51) Int. Cl.: A61F 7/08

(54) **Satz bestehend aus Bettflasche, Bettflaschenschraubverschluss und Bettflaschenöffner**
Kit consisting of hot water bottle, closure and opener
Kit composé d'une bouillotte, d'un verrou et d'un outil pour l'ouvrir

(30) Priorität: 26.05.1998 CH 115098
(43) Veröffentlichungstag der Anmeldung: 01.12.1999
(73) Patentinhaber: E. & H. Grob AG Kunststoffspritzerei, 9470 Buchs (CH)
(72) Erfinder: Grob, Hans-Jürg, 9470 Buchs (CH)
(74) Vertreter: Hasler, Erich, Dr.

(56) Entgegenhaltungen:
- EP-A- 0 242 198
- GB-A- 2 275 918
- US-A- 4 507 988
- US-A- 4 911 038
- US-A- 5 027 954

## Beschreibung

Die vorliegende Erfindung betrifft einen Satz bestehend aus einer Bettflasche mit einer in einem Einfülltrichter liegenden Einfüllöffnung mit Innengewinde, einem um eine Drehachse drehbaren Bettflaschenschraubverschluss und einen Bettflaschenöffner zum Öffnen der Bettflasche.

Obwohl die heutigen Wohnungen und Häuser in der Regel mit Zentralheizungen ausgerüstet sind und die Zimmer durch diese während der kalten Jahreszeit auf eine konstanten Raumtemperatur geheizt werden, erfreuen sich Bettflaschen nach wie vor bei jung und alt einer grossen Beliebtheit. Insbesondere bei Krankheit, wenn Fieber im Anzug ist, nimmt man gerne eine warme Bettflasche mit ins Bett, um dem Körper die nötige Wärme zuzuführen.

Die heutzutage gebräuchlichen Bettflaschen sind in der Regel aus einem elastomeren Kunststoff hergestellt. Um sich beim Befüllen der Bettflasche mit heissem Wasser die Hände nicht zu verbrennen, ist die Einfüllöffnung der Bettflasche mit einem trichterförmigen Kragen oder Einfülltrichter umgeben, welcher verhindert, dass Wasser, welches den Weg in die Einfüllöffnung nicht findet, nicht über die Bettflasche rinnt und die Hände der die Bettflasche füllenden Person verbrennt.

Die mit einem Innengewinde versehene Bettflaschenöffnung ist mit einem Bettflaschenschraubverschluss verschliessbar. Der Bettflaschenschraubverschluss ist ein mit einem Aussgewinde versehener Bolzen mit einem daran anschliessenden flachen Drehgriff zum Anfassen und Drehen desselben. Da eine Bettflasche möglichst keine harten Teile aufweisen soll, da diese als störend empfunden würden, ist der Bettflaschenschraubverschluss so ausgebildet, dass er innerhalb des Einfülltrichters liegt. Aus diesem Grund muss die Abmessung des Drehgriffs des Bettflaschenschraubverschlusses quer zur Drehachse kleiner als der kleinste Querschnitt des Einfülltrichters sein. Der kleine Drehgriff wiederum hat den Nachteil, dass durch dieses nur eine kleine Hebelwirkung ausgeübt werden kann. Da man aber möglichst ausschliessen möchte, dass heisses Wasser aus der Bettflasche austreten kann, ist man bestrebt, den Bettflaschenschraubverschluss möglichst stark anzuziehen. Dies wiederum führt dazu, dass anderntags, wenn die erkaltete Bettflasche geleert werden soll, der Bettflaschenschraubverschluss sich nur mit grosser Kraft lösen lässt. Dazu trägt auch bei, dass in der Bettflasche nach dem Erkalten des Wassers ein Unterdruck herrscht. Desweiteren sind sich viele Leute nicht sicher, auf welche Seite der Bettflaschenschraubverschluss zu drehen ist, um diesen herauszuschrauben.

Die EP-A-0 242 198 zeigt ein faltbares multifunktionelles Greifwerkzeug, welches u.a. als Flachzange genutzt werden kann. In den Griffen des Werkzeugs sind ein Flaschen/Dosenöffner, Messerklinge sowie ein Schraubenzieher untergebracht. Flaschen/ Dosenöffner, Messerklinge sowie Schraubenzieher können für den Gebrauch aus den Griffen herausgeschwenkt werden. Das Werkzeug der EP-A-0 242 198 ist ein Universalwerkzeug, welches in unterschiedlichsten Situationen Hilfe leisten soll.

Die US 4,507,988 zeigt einen Öffner für Getränkedosen und Flaschen. Das eine Ende ist gedacht zum Öffnen von Getränkedosen, welche eine mit dem Verschluss verbundene Lasche haben. Das andere Ende des Öffners erlaubt das Öffnen von Getränkeflaschen mit Drehverschlüssen.

Der Öffner für Getränkedosen besitzt zwei beabstandete, einander gegenüberliegende, offene obere und untere Backen. An der oberen Backe ist ein längliche Rippe angeordnet, welche in die Aussparung einer Lasche einrasten kann. Mit dem Getränkedosenöffner kann die eng an der Dosenoberseite liegende Lasche gefasst und angehoben werden. Die obere Backe des Öffners drückt beim Öffnen den Verschluss der Dose in das Doseninnere.

Es ist deshalb Aufgabe der vorliegenden Erfindung, die oben beschriebenen Nachteile des Stands der Technik möglichst zu beheben. Insbesondere sollen Mittel vorgeschlagen werden, welche es erlauben eine Bettflasche ohne grossen Kraftaufwand zu öffnen. Ein weiteres Ziel ist es, Mittel vorzuschlagen, welche einfach und sicher in der Anwendung und kostengünstig herstellbar sind.

Erfindungsgemäss wird die Aufgabe gelöst durch einen Satz bestehend aus einer Bettflasche mit einer in einem Einfülltrichter liegenden
Einfüllöffnung mit Innengewinde;
einem um eine Drehachse drehbaren Bettflaschenschraubverschluss mit flachem Drehgriff zum Verschließen der Bettflasche, und
einem Bettflaschenöffner mit
einem Kupplungsteil mit zwei in Abstand von einander angeordneten Vorsprüngen oder einem aus zwei beabstandeten Wänden gebildeten Schlitz zum Ankoppeln des Bettflaschenöffners an den flachen Drehgriff des Bettflaschenschraubverschlusses; und einem dem Kupplungsteil angeformten, beabstandeten Griffteil, welches sich, wenn der Bettflaschenöffner auf dem Bettflaschenschraubverschluss aufgesetzt ist, quer zur Drehachse erstreckt und eine solche Länge aufweist, dass die zum Öffnen des Bettflaschenschraubverschlusses nötige Kraft aufgrund der erzielbaren Hebelwirkung herabgesetzt ist; sowie
einem Verbindungsteil, welches parallel zur Drehachse verläuft und Kupplungsteil und Griffteil beabstandet und
miteinander verbindet.. Der Bettflaschenöffner hat den Vorteil, dass dieser zum Öffnen auf den Bettflaschenschraubverschluss aufsetzbar ist, wodurch einerseits das Problem der schlechten Zugänglichkeit des Bettflaschenschraubverschlusses beseitigt und andererseits durch den vergrösserten Hebelarm die zum Öffnen nötige Kraft herabgesetzt ist. Der Öffner ist vorzugsweise als Kunststoff-Spritzteil hergestellt.

Vorteilhaft ist die Längserstreckung des Hebels quer zur Drehachse so gewählt, dass die zum Öffnen des Bettflaschenschraubverschlusses nötige Kraft um wenigsten den Faktor 1.5, vorzugsweise um wenigsten den Faktor 2 herabgesetzt ist. Obwohl das Kupplungsteil beispielsweise lediglich zwei in Abstand voneinander angeordnete Vorsprünge aufweisen kann, welche den Bettflaschenschraubverschluss beim Drehen des Öffners in Eingriff nehmen können, ist gemäss einer bevorzugten Ausführungsform vorgesehen, dass die Vorsprünge gabelförmig sind. Die Gabeln des Öffners können den flachen Drehgriff des Bettflaschenschraubverschlusses in Eingriff nehmen. Diese Ausführung hat den Vorteil, dass der Bettflaschenöffner auf den Schraubverschluss aufsetzbar oder ansteckbar ist. Wenn die gabelförmigen Vorsprünge elastisch verformbar ausgebildet sind, kann der Bettflaschenöffner auf verschiedene Verschlüsse aufgesetzt werden, wobei der Öffner durch die Klemmwirkung der gabelförmigen Vorsprünge auf dem Verschluss gehalten sein kann.

Eine andere bevorzugte Ausführungsform sieht vor, dass das Kupplungsteil einen Schlitz zur Aufnahme des Drehgriffs eines Bettflaschenschraubverschlusses aufweist. Auch dieser Öffner ist zum Öffnen von verschieden Verschlüssen geeignet. Zweckmässigerweise sind das Kupplungsteil und der Hebel durch ein parallel zur Drehachse verlaufendes Verbindungsteil miteinander verbunden sodass der Hebel ausserhalb des Einfülltrichters der Bettflasche zu liegen kommt.

Gemäss einer besonders bevorzugten Ausführungsform ist der Hebel über eine Ratscheneinrichtung oder dergleichen mit dem Kupplungsteil verbunden. Das heisst, der Öffner ist zweiteilig, wobei die beiden Teile über eine Ratscheneinrichtung miteinander in Verbindung stehen. Die Ratscheneinrichtung ist vorzugsweise zwischen Verschliessen und Öffnen umschaltbar. Sie kann aber auch so ausgebildet sein, dass der Hebel in der einen Drehrichtung, d.h. zum Verschliessen der Flasche, leer dreht und in der Gegenrichtung das Kupplungsteil mitnimmt. Eine kostengünstige Variante sieht vor, dass der Hebel mittels einer Spiralfeder mit dem Kupplungsteil verbunden ist, wobei die Spiralfeder in Draufsicht gegen den Uhrzeigersinn gewickelt ist. Die Enden der Feder sind einerseits im Kupplungs- und anderseits im Griffteil fest verankert, z.B. durch Eingiessen. Bei Drehung des Griffteils im Gegenuhrzeigersinn kommt das Griffteil mit dem Kupplungsteil in Anschlag und nimmt letzteres mit. Bei Drehung des Griffteils im Uhrzeigersinn wird die Spiralfeder gegen die wirkende Federkraft aufgewickelt, und der Anwender merkt sofort, dass er die falsche Drehrichtung zum Öffnen der Bettflasche gewählt hat.

Grundsätzlich denkbar ist auch, dass der Bettflaschenöffner und der Schraubverschluss miteinander, z.B. formschlüssig, zusammenwirken können. Eine solche Variante sieht eine Aussparung im Griffteil des Schraubverschlusses vor, in welche das Kupplungsteil des Öffners eingreifen kann.

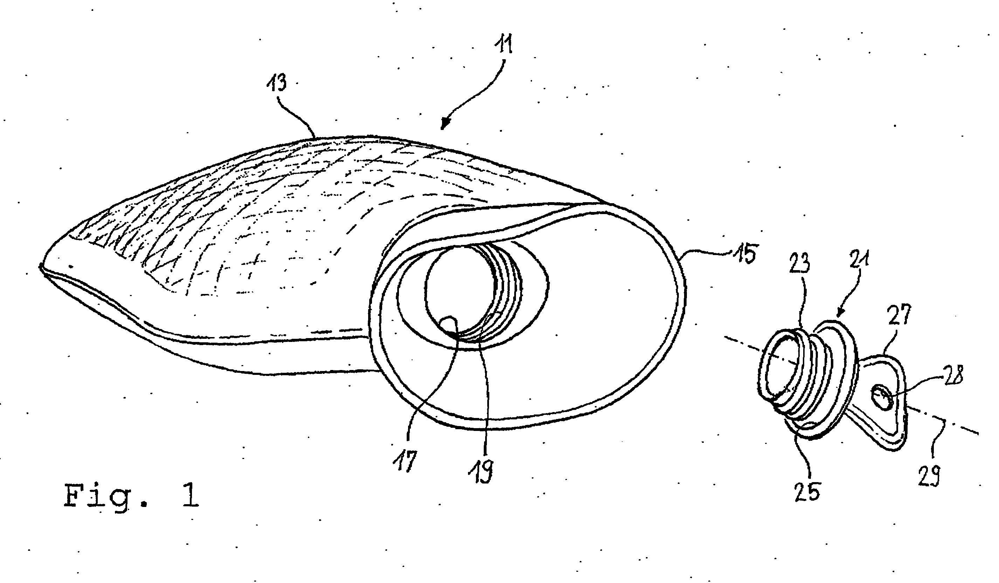

Es zeigt
- Figur 1:: eine perspektivische Ansicht einer bekannten Kunststoff-Bettflasche mit Schraubverschluss;
- Figur 2:: eine Vorderansicht einer ersten Ausführungsform eines Bettflaschenöffners;
- Figur 3:: eine Seitensicht des Öffners von Fig. 2;
- Figur 4:: eine Vorderansicht einer zweiten Ausführungsform eines Bettflaschenöffners;
- Figur 5:: eine Seitensicht des Öffners von Fig. 4;
- Figur 6:: eine Vorderansicht einer dritten Ausführungsform eines Bettflaschenöffners; und
- Figur 7:: eine Vorderansicht einer vierten Ausführungsform eines Bettflaschenöffners

Bekannte Kunststoff-Bettflaschen 11 besitzen einen Körper 13 aus einem elastomeren Kunststoff, an welchen ein Einfülltrichter 15 angeformt ist. Im Einfülltrichter 15 befindet sich eine Einfüllöffnung 17 mit einem Innengewinde 19, welche durch einen Schraubverschluss 21 verschliessbar ist. Der Schraubverschluss 21 besitzt ein Aussengewinde 23, welches mit dem Innengewinde 19 der Einfüllöffnung zusammenwirken kann. Eine Elastomerdichtung 25, welche am Drehgriff 27 des Schraubverschlusses 21 vorgesehen ist, dichtet die Bettflascheneinfüllöffnung bei eingedrehtem Schraubverschluss 21 ab.

Der Schraubverschluss 21 ist um eine Drehachse 29 drehbar. Im eingeschraubten Zustand ist der Drehgriff 27 des Schraubverschlusses 21 vom Einfülltrichter 15 umgeben. Aus diesem Grund sind die Abmessungen des Drehgriffs 27 begrenzt, und es kann mit diesem manuell nur ein bescheidenes Drehmoment ausgeübt werden. Dies ist die Ursache, dass es Leuten mit begrenzten physischen Kräften oftmals schwerfällt, einen fest verschrauben Bettflaschenverschluss wieder zu lösen. Eine öffnung 28 im Drehgriff 27 ermöglicht das separate Aufhängen des Verschlusses an einem spitzen Gegenstand.

Das erste Ausführungsbeispiel eines Bettflaschenöffners 31 besitzt ein Kupplungsteil 33, an welchem über ein mit der Referenznummer 42 bezeichnetes Verbindungsteil ein als Hebel wirkendes, trapezförmiges Griffteil 35 angeformt ist. Das Griffteil 35 besitzt einen umlaufenden Ringwulst 37 und in der Mitte eine dreieckige Aussparung 39, welche zum Aufhängen des Öffners 31 dienen kann. Das Kupplungsteil 33 weist einen Schlitz 41 auf, welcher durch zwei beabstandete Wände 43a,43b gebildet ist. Mittels des Schlitzes 41 ist der Öffner 31 auf den Drehgriff 27 eines Bettflaschenschraubverschlusses 21 aufsetzbar. Die Breite des Kupplungsteils 33 in der Ebene des Öffners entspricht ungefähr derjenigen des Drehgriffs 27 eines handelsüblichen Bettflaschenschraubverschlusses. Dadurch, dass das Griffteil 35 des Öffners 31 eine bedeutend grössere Hebelwirkung ermöglicht als der Drehgriff 27 des Bettflaschenschraubverschlusses 21, können auch fest zugeschraubte Bettflaschen mit dem Öffner 31 mühelos geöffnet werden.

Das Griffteil, welches beispielsweise als Drehhebel ausgebildet sein kann, ist über den Verbindungssteg mit dem Kupplungsteil verbunden. Der Verbindungssteg, welcher Teil des Griffes sein kann, sorgt dafür, dass das Griffteil, wenn dieses auf den Verschluss aufgesetzt ist, ausserhalb des Einfülltrichters zu liegen kommt, sodass der Öffner ungehindert betätigbar ist. Vorteilhaft sind Kupplungs-, Verbindungs- und Griffteil einstückig ausgebildet.

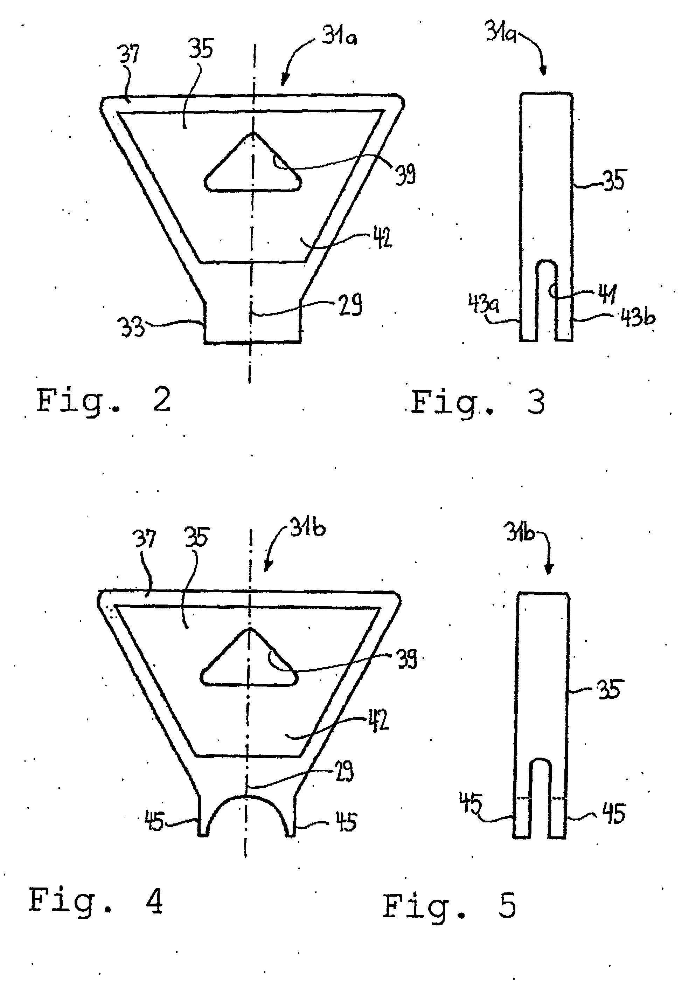

Das zweite Ausführungsbeispiel 31b der Figuren 4 und 5 unterscheidet sich vom ersten darin, dass das Kupplungsteil zwei in Abstand voneinander angeordnete Gabeln 45 aufweist, mit welchen der Drehgriff 27 eines Bettflaschenschraubverschlusses in Eingriff genommen werden kann.

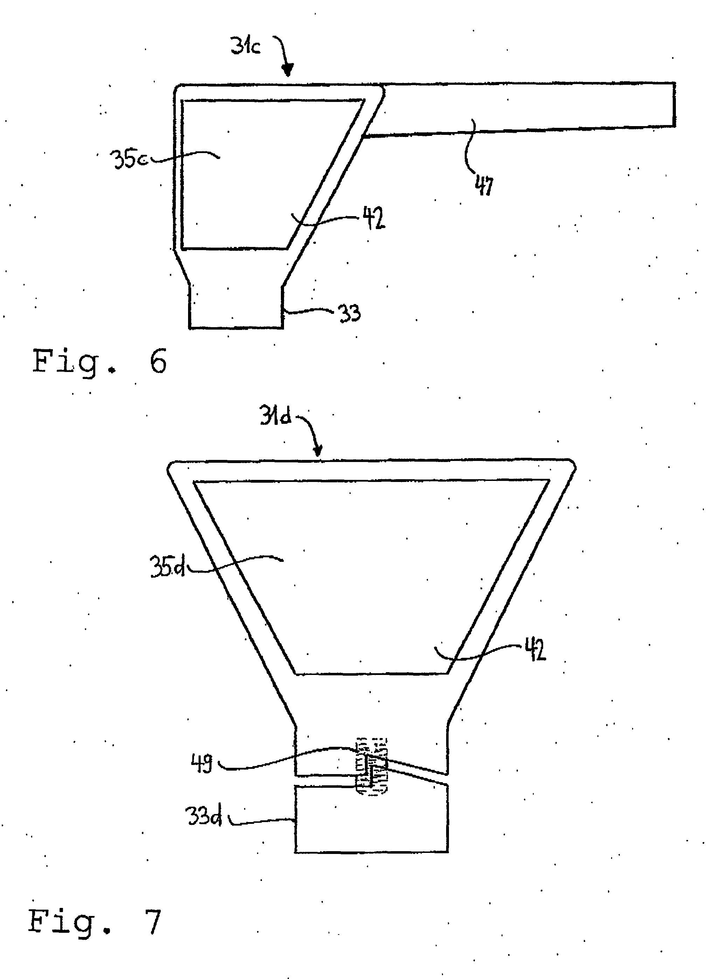

Das dritte Ausführungsbeispiel 31 c von Figur 6 ist asymmetrisch ausgebildet und besitzt am Griffteil 35c einen nach einer Seite hin stark verlängerten Hebelarm 47. Mit diesem Öffner kann ein sehr grosses Drehmoment ausgeübt werden.

Das vierte Ausführungsbeispiel 31 d unterscheidet sich von den oben beschriebenen darin, dass der Öffner 31d zweiteilig ist und das Griffteil 35d und das Kupplungsteil 33d mittels eines Federeinsatzes in Form einer Feder 49 miteinander in Verbindung stehen. Die Enden der Feder sind im Griff- resp. Kupplungsteil fest eingegossen. Obwohl ein kleiner Abstand zwischen dem Griff- und Kupplungsteil bestehen bleiben kann, stossen die beiden Teile vorzugsweise aneinander. Wird die Feder 49, welche in Draufsicht im Gegenuhrzeigersinn gewickelt ist, im Gegenuhrzeigersinn gedreht, so möchte sich diese zusammenziehen, wobei dann Kupplungs- und Griffteil in Anschlag gelangen und das Griffteil bei fortgesetzter Drehung des Griffteils ebenfalls mitgedreht wird. Ein solcher öffner hat den Vorteil, dass er nur zum Öffnen einer Bettflasche eingesetzt werden kann. Anstelle einer Feder 49 kann auch eine Ratscheneinrichtung eingesetzt werden.

Zum Öffnen einer Bettflasche wird der Bettflaschenöffner 31 auf den Bettflaschenschraubverschluss 27 aufgesetzt und dann im Gegenuhrzeigersinn gedreht, bis der Schraubverschluss gelöst ist. Nach dem Lösen kann dieser dann manuell ohne Öffner herausgedreht werden.

## Patentansprüche

1. Satz bestehend aus
einer Bettflasche mit einer in einem Einfülltrichter (15) liegenden Einfüllöffnung (17) mit Innengewinde (19);
einem um eine Drehachse (29) drehbaren Bettflaschenschraubverschluss (21) mit flachem Drehgriff (27) zum Verschließen der Bettflasche, und einem Bettflaschenöffner (31a-d) mit
einem Kupplungsteil (33, 31,45) mit zwei in Abstand von einander angeordneten Vorsprüngen oder einem aus zwei beabstandeten Wänden (43a,43b) gebildeten Schlitz (41) zum Ankoppeln des Bettflaschenöffners (31a-d) an den flachen Drehgriff (27) des Bettflaschenschraubverschlusses (21); und
einem dem Kupplungsteil (33,31,45) angeformten, beabstandeten Griffteil (35,47), welches sich, wenn der Bettflaschenöffner auf dem Bettflaschenschraubverschluss aufgesetzt ist, quer zur Drehachse (29) erstreckt und eine solche Länge aufweist, dass die zum Öffnen des Bettflaschenschraubverschlusses (21) nötige Kraft aufgrund der erzielbaren Hebelwirkung herabgesetzt ist; sowie
einem Verbindungsteil, welches parallel zur Drehachse verläuft und Kupplungsteil (33,31,45) und Griffteil (35,47) beabstandet und miteinander verbindet.

2. Satz nach Anspruch 1, **dadurch gekennzeichnet, dass** das Griffteil (35,47) eine solche Länge aufweist, dass die zum Öffnen des Bettflaschenschraubverschlusses (21) nötige Kraft um wenigsten den Faktor 1.5, vorzugsweise um wenigsten den Faktor 2 herabgesetzt ist.

3. Satz nach Anspruch 1 oder 2, **dadurch gekennzeichnet, dass** die Vorsprünge (45) gabelförmig sind, welche auf den Bettflaschenschraubverschluss (21) aufsetzbar sind.

4. Satz nach einem der Ansprüche 1 bis 3, **dadurch gekennzeichnet, dass** das Griffteil (35d) über eine Ratscheneinrichtung oder dergleichen mit dem Kupplungsteil verbunden ist.

5. Satz nach Anspruch 4, **dadurch gekennzeichnet, dass** das Griffteil (35d) mittels einer Spiralfeder (49) mit dem Kupplungsteil (33d) verbunden ist, sodass bei Drehung des Griffteils in der einen Drehrichtung das Kupplungsteil mitgedreht wird.

6. Satz nach einem der Ansprüche 1 bis 5, **dadurch gekennzeichnet, dass** das Kupplungsteil (33) Mittel aufweist, welche mit wenigstens einer Aussparung (28) am Drehgriff (21) des Bettflaschenschraubverschlusses zusammenwirken können.

## Claims

1. A set consisting of
a hot water bottle with a filling opening (17) with an internal screw thread (19) situated within a filling funnel (15);
a screw-in-type hot water bottle closure plug (21) with a flat turning grip (27) for closing the hot water bottle, the plug being capable of being rotated about an axis of rotation (29), and
a hot water bottle opener (31a-d) with
a coupling part (33, 31, 45) with two protrusions arranged at a distance from each other or a slot (41) formed by two walls (43a, 43b) set some distance apart for coupling the hot water bottle opener (31a-d) to the flat turning grip (27) of the screw-in-type hot water bottle closure plug (21); and
a grip part (35, 47) integrally attached to the coupling part (33, 31, 45) and separated from it that, when the hot water bottle opener is made to straddle the screw-in-type hot water bottle closure plug, extends at right angles to the axis of rotation (29) and is of such length that, due to the obtainable lever effect, the force needed for opening the screw-in-type hot water bottle closure plug (21) is reduced; and a connection part that extends parallel to the axis of rotation and separates the coupling part (33, 31, 45) and the grip part (35, 47) and also joins them to each other.

2. A set in accordance with Claim 1, **characterized in that** the grip part (35, 47) is of such length that the force needed for opening the screw-in-type hot water bottle closure plug (21) is reduced by at least the factor 1.5 and preferably by at least the factor 2.

3. A set in accordance with Claim 1 or Claim 2, **characterized in that** the protrusions (45) are forkshaped and can be made to straddle the screw-in-type hot water bottle closure plug (21).

4. A set in accordance with any one of Claims 1 to 3, **characterized in that** the grip part (35d) is connected to the coupling part by means of a ratchet device or similar.

5. A set in accordance with Claim 4, **characterized in that** the grip part (35d) is connected to the coupling part (33d) by means of a helical spring (49), so that whenever the grip part is rotated in the one direction of rotation, the coupling part will be rotated with it.

6. A set in accordance with any one of Claims 1 to 5, **characterized in that** the coupling part (33) is provided with means that can interact with at least one recess (28) in the turning grip (21) of the screw-in-type hot water bottle closure plug.

## Revendications

1. Set comprenant
une bouillotte avec une ouverture de remplissage (17) avec un filet intérieur (19) qui est située dans un entonnoir de remplissage (15) ;
une fermeture à vis de la bouillotte (21) rotative autour d'un axe de rotation (29) avec une poignée tournante plate (27) pour fermer la bouillotte et
un ouvre-bouillotte (31 a-d) avec
une pièce d'accouplement (33, 31, 45) avec deux saillies placées espacées l'une de l'autre ou une fente (41) formée par deux parois espacées (43a, 43b) pour coupler l'ouvre-bouillotte (31 a-d) à la poignée tournante plate (27) de la fermeture à vis de la bouillotte (21) et
une partie poignée (35, 47) espacée moulée à la pièce d'accouplement (33, 31, 45) qui s'étend transversalement à l'axe de rotation (29), lorsque l'ouvre-bouillotte est placé sur la fermeture à vis de la bouillotte, et qui présente une longueur telle que la force nécessaire à l'ouverture de la fermeture à vis de la bouillotte (21) est diminuée en raison de l'effet de levier qui peut être obtenu ainsi
qu'une pièce de raccord qui va parallèlement à l'axe de rotation et qui espace la pièce d'accouplement (33, 31, 45) et la partie poignée (35, 47) et qui les relie l'une à l'autre.

2. Set selon la revendication 1, **caractérisé en ce que** la partie poignée (35, 47) présente une longueur telle que la force nécessaire à l'ouverture de la fermeture à vis de la bouillotte (21) est diminuée d'au moins le facteur 1,5, de préférence d'au moins le facteur 2.

3. Set selon la revendication 1 ou 2, **caractérisé en ce que** les saillies (45) sont en forme de fourches qui peuvent être placées sur la fermeture à vis de la bouillotte (21).

4. Set selon l'une des revendications 1 à 3, **caractérisé en ce que** la partie poignée (35d) est reliée à la pièce d'accouplement par un dispositif à cliquet ou équivalent.

5. Set selon la revendication 4, **caractérisé en ce que** la partie poignée (35d) est reliée à la pièce d'accouplement (33d) au moyen d'un ressort en spirale (49) si bien que, lors de la rotation de la partie poignée dans l'un des sens de rotation, la pièce d'accouplement est tournée en même temps.

6. Set selon l'une des revendications 1 à 5, **caractérisé en ce que** la pièce d'accouplement (33) présente des moyens qui peuvent coopérer avec au moins une réservation (28) sur la partie poignée (21) de la fermeture à vis de la bouillotte.
